# EUROPEAN PATENT APPLICATION

(11) **EP 2 348 102 A1**
(43) Date of publication of application: **27.07.2011**
(21) Application number: 11151259.6
(22) Date of filing: 18.01.2011
(51) Int. Cl.: C12N 1/20, A61K 35/74, A23L 1/03, C12R 1/225

(54) **Lactobacillus paracasei strain PC201, and compositions and uses thereof**

(30) Priority: 22.01.2010 IT MI20100081
(71) Applicant: Farmapros S.p.A., 47891 Dogana (SM)
(72) Inventor: Guasti, Pierluigi, 47924 Rimini (RN) (IT); Agostini, Alida, 59100 Prato (PO) (IT); Bottazzi, Vittorio, 59100 Prato (PO) (IT); Rebecchi, Annalisa, 59100 Prato (PO) (IT)
(74) Representative: Minoja, Fabrizio

(57) **Abstract**

Disclosed is *Lactobacillus paracasei* strain PC201, deposited at the Pasteur Institute's Collection Nationale de Cultures de Microorganismes (CNCM) under access number "CNCM I-4214" on 28 July 2009 pursuant to the Budapest Treaty.

The present invention also relates to compositions containing said *Lactobacillus paracasei* strain PC201 and to the uses of said *Lactobacillus paracasei* strain PC201.

## Description

### Technical field of invention

The present invention relates to a strain of *Lactobacillus paracasei,* in particular called PC201.

The present invention also relates to compositions containing said strain and the corresponding uses of said strain.

### Prior art

The term "probiotics" indicates those micro-organisms which, when ingested in sufficient quantities, reach the intestine alive and active and perform functions beneficial to the body.

Probiotics have been widely used in both the medical and the dietary fields for a long time.

Foods/supplements with probiotics are foods which contain a sufficiently large number of live, active probiotic micro-organisms able to reach the intestine, multiply, and performing a balancing action on the intestinal microflora. Said foods are consequently able to promote and improve the bodily functions.

Probiotics have been widely used in the pharmaceutical industry, and are present in numerous formulations for the treatment of intestinal disorders caused by the presence of pathogens, for example, or as adjuvants during or at the end of antibiotic treatments.

The micro-organisms most commonly used in probiotic preparations belong to the genera *Bifidobacterium* and *Lactobacillus.*

Lactobacilli (milk enzymes) play a crucial role in regulating the intestinal microflora, by producing lactic acid and specific antibacterial substances.

Lactobacilli are involved in numerous metabolic activities, such as deconjugation of bile acids, breakdown of nitrosoamines, neutralisation of bacterial toxins and anticarcinogenic activity, which are particularly important in maintaining good health and preventing the onset of various disorders.

The division of probiotic species of *Lactobacillus* into *Lactobacillus acidophilus, Lactobacillus casei, Lactobacillus reuteri-fermentum* and *Lactobacillus plantarum* is currently accepted as reliable.

The following species of *Lactobacillus casei* can be identified: *Lactobacillus casei, Lactobacillus rhamnosus* and *Lactobacillus paracasei.*

It has been established by the scientific community that in order to be classified as a probiotic strain, a micro-organism must possess certain characteristics, some of which are described below.

In particular, the strain must be characterised by resistance to technological processes and resistance to gastric acid and bile.

The micro-organism must be able to adhere to the intestinal epithelium and persist, albeit for short periods, in the gastrointestinal tract.

Transient colonisation of the intestinal epithelium gives the individual resistance to colonisation by, and consequent proliferation of, pathogenic bacteria (various pathogenic bacteria lose their virulence at the same time as their ability to adhere to the mucous membranes).

In general, all strains of *L. paracasei* tolerate low A_{w} (Water Activity), heating to 60°C for 30 minutes, and a pH of 3.0.

*Lactobacillus paracasei* is a homofermentative species of the Lactobacillus group, frequently found as an NSLAB (non-starter) culture in cheeses with a medium and long ripening period, and is also a probiotic which is relatively resistant to the conditions in the gastric environment.

However, new strains of *Lactobacillus,* especially *paracasei,* need to be identified which are more resistant than the strains currently known, to ensure their adaptability and survival under environmental conditions and the physiological and pathological conditions which may occur during the transit through the gastrointestinal apparatus.

It is therefore necessary to identify new preparations based on milk enzymes with their own natural resistance.

### Summary of the invention

The present invention relates to a strain of *Lactobacillus paracasei* called PC201, deposited at the Pasteur Institute's Collection Nationale de Cultures de Microorganismes (CNCM) under access number "CNCM I-4214" on 28 July 2009.

The present invention also relates to compositions containing *Lactobacillus paracasei* strain PC201 and at least one suitable carrier.

The invention also relates to *Lactobacillus paracasei* strain PC201 for use as a medicament/nutraceutical and the use of *Lactobacillus paracasei* strain PC201 to prepare a composition for the prevention and treatment, or as an adjuvant to the prevention and treatment, of gastroenteric diseases and/or gastroenterological symptoms not associated with disease.

### List of figures

Figures 1a, 1b and 1c show the genetic identification of *Lactobacillus paracasei* PC201.
Figure 2 shows a scanning electron microscope (SEM) image of *Lactobacillus paracasei* PC201 after 24 hours' incubation at 30°C in MRS.
Figure 3 shows a scanning electron microscope (SEM) image of the stage of detachment of the natural protective capsule during the process of autolysis of *Lactobacillus paracasei* PC201.
Figure 4 is a scanning electron microscope (SEM) image of *Lactobacillus paracasei* PC201, which shows the residues of the capsule shell during the process of autolysis.

### Detailed description of the invention

The present invention relates to a strain of *Lactobacillus paracasei.*

The strain of *Lactobacillus paracasei* isolated and named PC201 was deposited at the Pasteur Institute's Collection Nationale de Cultures de Microorganismes (CNCM) under access number "CNCM I-4214" on 28 July 2009 pursuant to the Budapest Treaty.

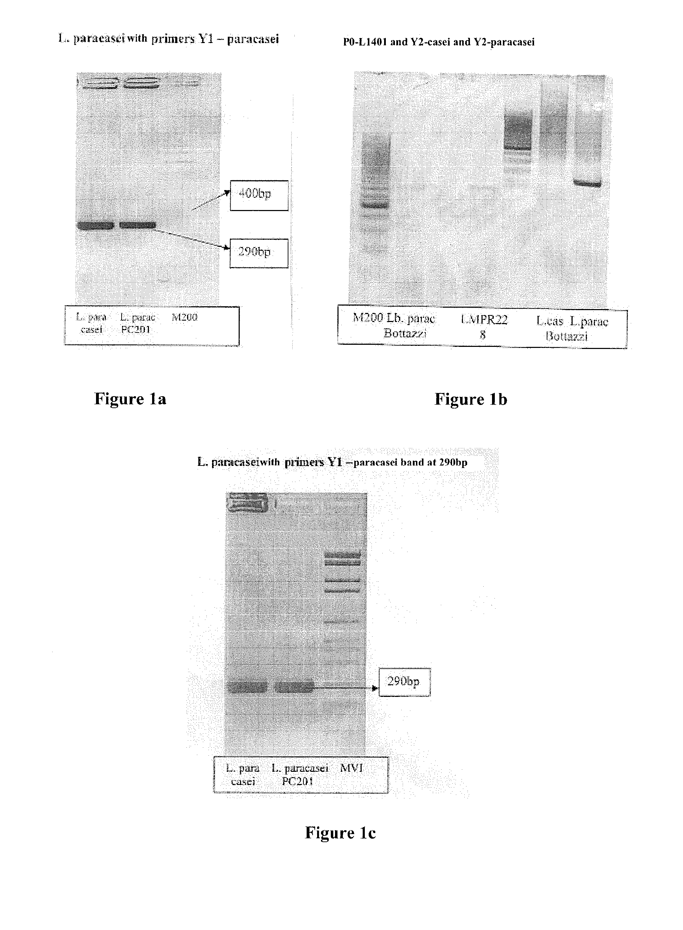

Strain PC201 (CNCM 1-4214) has been identified, in terms of taxonomy, as a strain of the species *Lactobacillus paracasei* on the basis of ribosomal RNA sequence 16S (Figs. 1a, 1b, 1c). In particular, the genetic identification was performed according to the method described by Ward L.J.H. and Timmins M.J., Differentiation of Lactobacillus casei, Lactobacillus paracasei and Lactobacillus rhamnosus by polymerase chain reaction, Letters Appl. Microbiol., 29, 90-92 (1999), with the development of polymerase chain reaction primers which are specific to each of the above-mentioned species, and based on the differences in region V1 of rRNA sequence 16S.

The primers used to identify the species *L. paracasei* are para-Y2, as reported in the literature.

It has now surprisingly been found that strain PC201 possesses a protective covering, namely a natural protective capsule, and can therefore can be considered "naturally coated".

Strain PC201 is characterised by units consisting of a capsule comprising two cells per unit (bicellular capsule); each capsular protective unit represents a bicellular structure because it encloses two cells at a time.

Said capsule forms when the culture is reproducing, and presents as a rigid sac-shaped structure, which acts as a very uniform coating that plays a crucial role in protecting the cells throughout the cell life, and also keeps the cells connected to one another to form the filaments typical of streptobacilli.

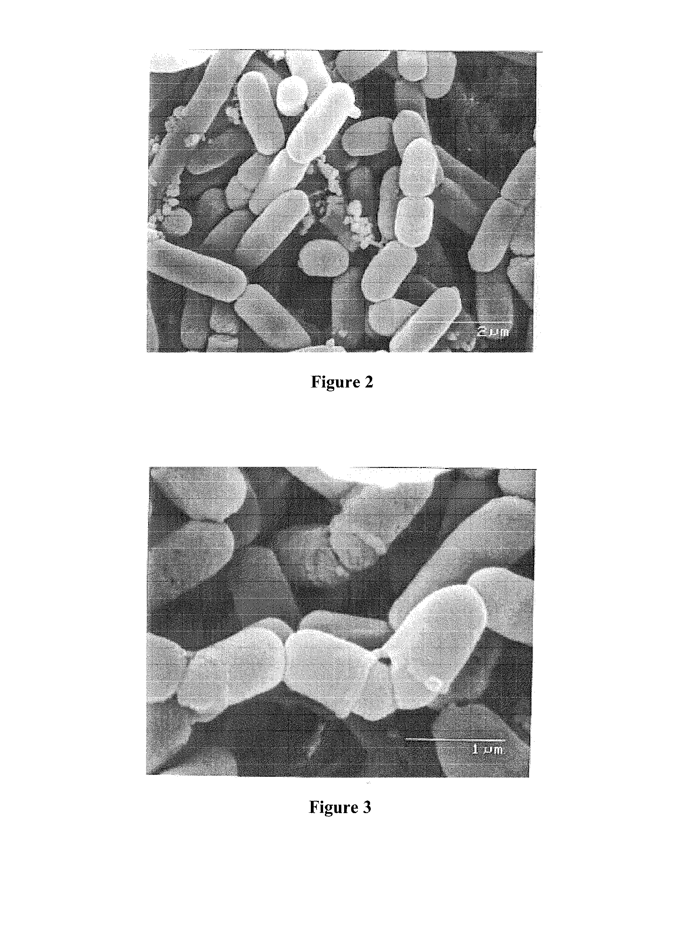

Observation under the scanning electron microscope (Fig. 2) of *Lactobacillus paracasei* PC201 after 24 hours' incubation at 30°C in MRS substrate shows the typical arrangement of cells with bacillar morphology forming filaments of variable lengths (streptobacilli).

Scanning electron microscope (SEM) images show, for strain PC201, the natural process of cell microencapsulation with a protective sheath, forming a natural capsule, which is synthesised during the cell development of the micro-organism.

The SEM comparison between *L. paracasei* strain PC201 and other strains of the same species demonstrates that all cells of strain PC201 have a protective covering (natural capsule). Said capsule is always well developed, structured and thick.

The capsular unit also contains two cells, which are protected by the capsule; this is evident when the cell undergoes lysis, and has never been observed in other strains of *L. paracasei.*

In the other strains of *L. paracasei,* not all the cells have a natural coating; however, in the cases where a protective coating is present, it is always much less developed, thinner and less structured, and does not constitute a true capsular sac.

A natural coating forming bicellular units is never observed in the other strains, whose structure is always unicellular; a protective coating containing two cells has never been observed in any case.

Moreover, in the other strains, during division, the coating divides together with the cell, and the process of cell division takes place by simple binary fission, as in all bacteria.

After a certain period of time, the capsule of strain PC201 is destroyed by autolysis; however, this process is lengthy and influenced by various factors which act together or individually.

By analysing SEM images, at least six stages forming the basis of the autolysis process of *Lactobacillus paracasei* PC201 can be identified, as follows:
1. start of capsule lesion;
2. circularisation of capsule break line;
3. formation of a capsular ring;
4. detachment of sections of the rigid capsule, with the evident presence of two bacillary cells per capsular unit;
5. emptying of the capsules, with the presence of capsular residues which are still rigid;
6. lysis of the capsular coverings and the *Lactobacillus paracasei* PC201 cell.

The autolysis process begins with the appearance of a lesion halfway along the length of the natural capsule, exactly at the septum joining the two cells contained by each capsule (bicellular capsule), although the capsular unit always maintains the general structure of streptobacilli.

The initial lesion gradually becomes circular, the capsule contracts, and the edge of the break line acquires the appearance of a ring thicker than the capsule.

Finally, the capsule contracts further and at the same time divides into two parts, revealing the two cells of the capsular unit which were previously hidden.

Figure 3 shows a detail of the mechanics of the process of detachment of the protective capsule: during the detachment of the two sections of the protective capsule the two cells, previously encased and protected, frequently move from an aligned position to a V-shape. This change of spatial configuration mechanically facilitates the release of both cells contained in the unit.

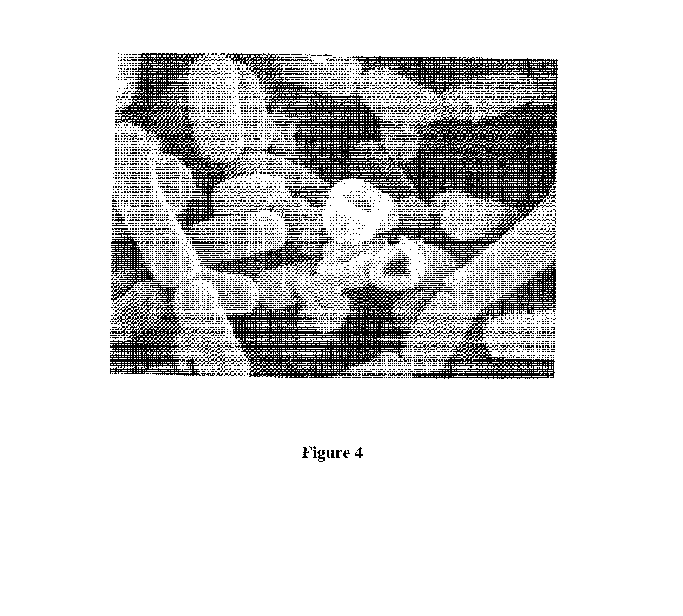

Figure 4 shows the residues of the capsular casing which is now empty, but still retains its original natural architectural structure.

As the protective casing opens very slowly, the process of lysis takes much longer than the formation of the capsular unit.

The resistance of the natural capsule is always high, even under accentuated conditions of environmental stress.

The long time required to complete the autolysis process means that the live cells contained in the bicellular capsules are preserved for a long time.

Some advantages associated with the particular structure of *Lactobacillus paracasei* strain PC201 are greater stability of the product as raw material, and consequently greater stability of the finished product containing it.

These characteristics also increase the viability and resistance of strain PC201 when administered, for example, at gastroenteric level, if administered orally, and increase the possibility of transient colonisation of the intestine and the possibility of exerting beneficial effects, for example on humans.

*Lactobacillus paracasei* strain PC201 possesses antibiotic resistance comparable to that of the other known strains of *L. paracasei.*

In a preferred embodiment, *Lactobacillus paracasei* PC20 is in powder form, and even more preferably in freeze-dried form.

According to a preferred aspect, *Lactobacillus paracasei* PC201 can be formulated in compositions containing at least one suitable carrier.

These compositions can also include, for example, other bacterial species, yeasts, prebiotics, vitamins, minerals, plant extracts, nutritional ingredients, etc.

According to a further aspect, strain PC201 can be administered in suitable compositions containing an effective number of viable cells, preferably freeze-dried, in the form of soft capsules; rigid capsules; tablets; pills; granulates; microgranulates; powders; or in liquid form, such as solutions or suspensions in water or in other liquids; drops; syrups; creams; gels; emulsions; lotions; sprays; pessaries; etc.

If a micro-organism is to be used as lactic enzyme in preparations which meet the requirements for long storage, a very important characteristic is its content of enzymes which govern the cell autolysis process, and its sensitivity to external environmental factors which act on biological structures unprotected by isolation barriers.

Hence the micro-organism *Lactobacillus paracasei* PC201 can be particularly indicated and useful in preparations for which lengthy storage under common environmental conditions is required, in particular in the preparation of bacterial biomasses.

Strain PC201 can be used in the medical field, as a medicament or medical device, or in the dietary or cosmetic field, for either human or veterinary use.

According to a preferred aspect, strain PC201 can be administered orally or topically.

Moreover, *Lactobacillus paracasei* strain PC201 can be used to prepare a composition for the prevention and treatment, or as adjuvant to the prevention and treatment, of gastroenteric disorders (such as intestinal dysbiosis, in particular the types involved in the pathogenesis and/or progress of disorders like IBS or irritable bowel syndrome, diarrhoea of various origins, ulcerating colitis, other disorders and/or alterations of the gastroenteric physiology) and/or of gastroenterological symptoms not associated with disease (such as functional constipation in the elderly and constipation caused by climate changes/travel).

*Lactobacillus paracasei* PC201 can preferably be administered in conjunction with or after antibiotic treatment.

According to a preferred aspect, strain PC201 can be used in food preparations, such as yoghurt, dairy products, diet supplements, foods destined for special diets, etc.

The examples set out below further illustrate the invention, by way of example but not of limitation.

### Examples

### 1. Formulation examples

### 1.1. Formulation 1

A formulation having the composition shown in Table I in the form of a 3,000 mg sachet is prepared from powdered ingredients according to known procedures.

A freeze-dried culture of *Lactobacillus paracasei* PC201 at the concentration of 250 CFU/g is used to prepare the product.

The lactobacilli are stored in the freezer (at T= -18°C). The lactobacilli required for a preparation are transferred to the refrigerator the night before production and left out of the refrigerator before preparation for as long as necessary to reach ambient temperature.

**Table I - Formulation 1**

| **Ingredient** | **Composition (mg)** |
|---|---|
| *L. paracasei* PC201 | 36.00 |
| Zinc gluconate | 71.43 |
| Maltodextrins | 2,892.57 |
| Total | 3,000.00 |

### 1.2. Formulation 2

A formulation having the composition shown in Table II in the form of a 3,700 mg sachet is prepared from powdered ingredients according to known procedures.

A freeze-dried culture of *Lactobacillus paracasei PC201* at the concentration of 250 CFU/g is used to prepare the product.

The lactobacilli are stored in the freezer (at T= -18°C). The lactobacilli required for a preparation are transferred to the refrigerator the night before production and left out of the refrigerator before preparation for as long as necessary to reach ambient temperature.

**Table II - Formulation 2**

| **Ingredient** | **Composition (mg)** |
|---|---|
| *L. paracasei* PC201 | 48 |
| Fructo-oligosaccharides | 2,500 |
| Maltodextrins | 1,152 |
| Total | 3,700 |

### 1.3. Formulation 3

A formulation having the composition shown in Tables IIIa and IIIb in the form of single-dose vials with measuring cap is prepared according to known procedures from powdered ingredients (except for water and sodium lactate solution). In particular, the solvent phase has the composition shown in Table IIIa, while the powder contained in the measuring cap has the composition shown in Table IIIb.

A freeze-dried culture of *Lactobacillus paracasei PC201* at the concentration of 250 CFU/g is used to prepare the product.

The lactobacilli are stored in the freezer (at T= -18°C). The lactobacilli required for a preparation are transferred to the refrigerator the night before production and left out of the refrigerator before preparation for as long as necessary to reach ambient temperature.

**Table IIIa - Formulation 3**

| **Ingredient** | **Composition (mg)** |
|---|---|
| Fructose | 2,100 |
| Acacia honey | 700 |
| Fructo-oligosaccharides | 700 |
| Potassium sorbate | 13.4 |
| Sodium lactate 60% | |
| solution | 50 |
| Cream flavouring | 3.5 |
| Anhydrous citric acid | q.s. for pH 3.9 |
| Water | q.s. for 10 ml |

**Table IIIb - Formulation 3**

| *Ingredient* | Composition (mg) |
|---|---|
| Brewer's yeast | 192 |
| *L. paracasei* PC201 | 24 |
| Nicotinamide | 5.02 |
| Calcium pantothenate | 2.33 |
| Riboflavin | 1.02 |
| Pyridoxine hydrochloride | 0.86 |
| Thiamine hydrochloride | 0.82 |
| Cyanocobalamin 0.1% | 0.7 |
| Total | 226.75 |

### 1.4. Formulation 4

**Table IV - Formulation 4**

| **Ingredients** | **Composition mg/sachet** |
|---|---|
| Maltodextrins | 300.00 |
| Fructo-oligosaccharides (FOS) | 1,000.00 |
| *L. paracasei* PC201 | 36.00 |
| Zinc gluconate | 57.14 |
| **TOTAL** | 1,393.14 |

A formulation having the composition shown in Table IV in the form of a 1,393.14 mg sachet is prepared from powdered ingredients according to known procedures.

A freeze-dried culture of *Lactobacillus paracasei PC201* at the concentration of 250 CFU/g is used to prepare the product.

The lactobacilli are stored in the freezer (at T= -18°C). The lactobacilli required for a preparation are transferred to the refrigerator the night before production and left out of the refrigerator before preparation for as long as necessary to reach ambient temperature.

### 1.5. Formulation 5

**Table V - Formulation 5**

| **Ingredients** | **mg/sachet** |
|---|---|
| Fructose | 1,648.335 |
| Magnesium citrate | 1,686.625 |
| Zinc citrate trihydrate | 32.04 |
| Dibasic calcium phosphate | 33.78 |
| L-cysteine base | 50 |
| Pyridoxine hydrochloride | 1.22 |
| *L. paracasei* PC201 | 48 |
| **TOTAL** | 3,500 |

A formulation having the composition shown in Table V in the form of a 3,500 mg sachet is prepared from powdered ingredients according to known procedures.

A freeze-dried culture of *Lactobacillus paracasei* PC201 at the concentration of 250 CFU/g is used to prepare the product.

The lactobacilli are stored in the freezer (at T= -18°C). The lactobacilli required for a preparation are transferred to the refrigerator the night before production and left out of the refrigerator before preparation for as long as necessary to reach ambient temperature.

### 2. Bioassays

### 2.1. Test for survival of different strains of Lactobacillus paracasei under different growth conditions

Survival tests were conducted on 4 different strains of *L. paracasei* (in particular PC201, *L. paracasei* isolated from acid serum, *L. paracasei* from commercial yoghurt, and *L. paracasei* from a commercial diet supplement under different growth conditions).

The ability of the strain to withstand the following conditions was tested:
- the action of the gastric juices (pH = 2.5 and 3),
- the action of pancreatic juice in the intestine (pH=8),
- the presence of bile salts (0.3% oxgall),
factors usually considered among the requirements of probioticity (see: A Bezkorovainy, Probiotics: determinants of survival and growth in the gut Am J Clin Nutr 2001;73(suppl):3995-405S; C. Pennacchia, D. Ercolini, G. Blaiotta, O. Pepe, G. Mauriello, F. Villani. Selection of Lactobacillus strains from fermented sausages for their potential use as probiotics Meat Science 67 (2004) 309-317; W.P. Charteris, P.M. Kelly, L. Morelli and J.K. Collins. Development and application of an in vitro methodology to determine the transit tolerance of potentially probiotic Lactobacillus and Bifidobacterium species in the upper human gastrointestinal tract. J Appl. Microbiol. 84, (1998) 759-768.).

The strain *L. paracasei* PC201 was revitalised, as were the other strains used in the tests, by means of two passages in MRS broth. The test at pH 2.5 and 3 was conducted in two test tubes each containing 10 ml of MRS culture broth, adjusted to pH 2.5 and 3 respectively with 1N HCl. The test tubes were then inoculated with 1% of a culture and incubated overnight at 37°C. At time t=0 and after 1h, 2h and 3h, samples were taken for the plate count using MRS agar as culture medium, and the plates were anaerobically incubated at 37°C for 48 hours.

The test according to the procedure described above was also conducted at pH 8.

The test of sensitivity to bile salts was conducted in 10 ml of MRS with the addition of 0.3% oxgall (Difco). In this case, in view of the longer transit through the intestine, samples were taken at time t=0 and after 1h, 2h and 4h. The culture was incubated and the plate count performed under the same conditions as described above.

The results demonstrate that in MRS at pH 8, in the presence of 0.3% oxgall, the strain survives and even multiplies, doubling the CFU/ml value after 4 hours.

Tables VIa, VIb, VIc and VId summarise the data obtained for the various strains tested under the different experimental conditions described above.

**Table VIa**

| ***L**. paracasei* **PC201** | **CFU/ml** | | | | |
|---|---|---|---|---|---|
| | T0h | T1h | T2h | T3h | T4h |
| MRS | 1.6 x 10⁷ | | | | 5.7 x10⁷ |
| MRS pH 8 | 1.6 x 10⁷ | 1.9 x 10⁷ | 3 x 10⁷ | | 4.7 x 10⁷ |
| MRS pH 3 | 1.6 x 10⁷ | 1.9 x 10⁷ | | 2.1 x 10⁷ | |
| MRS pH 2,5 | 1.6 x 10⁷ | 8.4 x 10⁶ | 6.8 x 10⁵ | 8.6 x 10⁴ | |
| MRS+ 0.3% oxgall | 1.6 x 10⁷ | 3 x 10⁷ | 3.1 x 10⁷ | | 4.4 x 10⁷ |

**Table VIb**

| ***L. paracasei* isolated from acid serum** | CFU/ml | | | | |
|---|---|---|---|---|---|
| | T0h | T1h | T2h | T3h | T4h |
| MRS | 5 x 10⁶ | | | | 2.2 x 10⁷ |
| MRS pH 8 | 5 x 10⁶ | 3.6 x 10⁶ | 1.2 x 10⁶ | | 8.4 x 10⁵ |
| MRS pH 3 | 5 x 10⁶ | 1.2 x 10⁷ | 1.5 x 10⁷ | 1.8 x 10⁷ | |
| MRS pH 2.5 | 5 x 10⁶ | 1.1 x 10⁷ | 1.1 x 10⁷ | 9.7 x 10⁶ | |
| MRS+ 0.3% oxgall | 5 x 10⁶ | <10⁵ | <10⁵ | | <10⁵ |

**Table VIc**

| ***L. paracasei* isolated from commercial yoghurt** | CFU/ml | | | | |
|---|---|---|---|---|---|
| | T0h | T1h | T2h | T3h | T4h |
| MRS | 1.7 x 10⁷ | | | | 2.9 x 10⁷ |
| MRS pH 8 | 1.7 x 10⁷ | 1.4 x 10⁷ | 2.2 x 10⁷ | | 2.2 x 10⁷ |
| MRS pH 3 | 1.7 x 10⁷ | 1.4 x 10⁷ | 1.1 x 10⁷ | 1.0 x 10⁷ | |
| MRS pH 2.5 | 1.7 x 10⁷ | <10⁵ | <10⁵ | <10⁵ | |
| MRS + 0.3% oxgall | 1.7 x 10⁷ | 1.0 x 10⁵ | 1.0 x 10⁵ | | 1.0 x 10⁵ |

**Table VId**

| ***L. paracasei* isolated from commercial diet supplement** | **CFU/ml** | | | | |
|---|---|---|---|---|---|
| | T0h | T1h | T2h | T3h | T4h |
| 1 % MRS inoculation | 3.7 x 10⁷ | | | | 8.1 x 10⁷ |
| MRS pH 8 | 3.7 x 10⁷ | 3.5 x 10⁷ | 4.6 x 10⁷ | | 6.1 x 10⁷ |
| MRS pH 3 | 3.7 x 10⁷ | 9.8 x 10⁶ | | 9.9 x 10⁶ | |
| MR 2.5 | 3.7 x 10⁷ | 1.0 x 10⁶ | 5 x 10⁵ | 7.4 x 10⁴ | |
| MRS + 0.3% oxgall | 3.7 x 10⁷ | 3.1 x 10⁶ | 1.4 x 10⁶ | | 5.2 x 10⁵ |

### Findings:

- *L. paracasei* PC 201 is resistant to pH 8 and pH 3.

At pH 2.5, only some of the cells survive, and there was a reduction of approx. one log per hour's residence at pH 2.5; the lactobacilli showed a reduction exceeding 2 logs after 3 hours.

In the presence of bile salts (0.3% oxgall), *L. paracasei* PC 201 not only resists, but develops.
- *L. paracasei* isolated from acid serum exhibits resistance and weak growth at pH 3 and pH 2.5.

At pH 8 only some of the cells survive, and are reduced by approx. 0.5 log after 4 hours.

In the presence of bile salts (0.3% oxgall), *L. paracasei* isolated from acid serum was reduced by 1.5 log after only 1 hour.
- *L. paracasei* isolated from commercial yoghurt exhibited resistance to pH 8 and pH 3, whereas at pH 2.5, it was reduced by approx. 2 logs after only 1 hour.

In the presence of bile salts (0.3% oxgall), the lactobacillus was reduced by 2 logs after only 1 hour, and the reduction remained constant until the 3h sample was taken.
- *L. paracasei* isolated from a commercial diet supplement showed resistance at pH 8 and pH 3 with behaviour similar to *L. paracasei* PC 201, whereas at pH 2.5 it underwent a reduction with a similar pattern to *L. paracasei* PC 201.

In the presence of bile salts (0.3% oxgall), it underwent a reduction of 1 log after only 1 hour, and approx. 2 logs after 3 hours.

To sum up, *L. paracasei* PC 201 exhibits greater overall resistance to the different growth conditions than the other *L. paracasei* strains tested; in particular, it exhibits greater resistance to bile salts than the other *L. paracasei* strains.

### 2.2. Test of survival of L. paracasei PC201 in liquid culture at different storage temperatures

Two cultures of *L. paracasei* developed overnight (PC201 and from a commercial diet supplement) were stored in their growth medium (pH 3.6) at 4°C and 25°C, and samples were taken periodically (at time=0, 7 days, 15 days, 21 days and 35 days) to evaluate the survival of the strain with a plate count on MRS medium with the appropriate dilutions. Table VII summarises the survival data obtained for the two different strains.

**Table VII**

| | **CFU/ml** | | |
|---|---|---|---|
| **(MRS growth medium pH 3.6)** | | **4°C** | **25°C** |
| ***L. paracasei* PC201** | | | |
| **T0** | 1.2 x 10⁹ | | |
| **T 7gg** | | 1.1 x 10⁹ | 5.5 x 10⁸ |
| **T 15gg** | | 6.4 x 10⁸ | 4.6 x 10⁷ |
| **T 21gg** | | 4.8 x 10⁸ | 5.8 x 10⁶ |
| **T 35gg** | | 6.2 x 10⁶ | 9 x 10⁵ |
| | | | |

| | **CFU/ml** | | |
|---|---|---|---|
| **(MRS growth medium pH 3.6)** | | **4°C** | **25°C** |
| ***L. paracasei* commercial diet supplement** | | | |
| **T0** | 1.7 x 10⁹ | | |
| **T7gg** | | 1.2 x 10⁹ | 4.2 x 10⁸ |
| **T 15gg** | | 7.4 x 10⁸ | 4.7 x 10⁷ |
| **T 21gg** | | 5.1 x 10⁸ | 6.2 x 10⁶ |
| **T 35gg** | | 6 x 10⁶ | 8.8 x 10⁵ |

In this case, the behaviour of the two strains of *L. paracasei* was similar, ie. up to 21 days the strains were reduced by approx. half a log at 4°C and over 2 logs at 25°C. After 35 days the reduction was evident under both growth conditions for both strains. These data are explained by the acidity of the culture broth.

### 2.3. Method used for count of freeze-dried L. paracasei PC 201

1 g of lyophilisate was diluted in 9 ml of saline solution. The successive dilutions were obtained by transferring 1 ml of diluate into 9 ml of saline solution contained in the next test tube. The sample was vortexed every time. Next, 100 µl of the 3 dilutions considered most suitable to exhibit a number of colonies sufficient for the count were deposited on the surface of each plate containing agarised MRS. The sample was distributed uniformly all over the surface with a sterile spatula. Finally, the plates were incubated anaerobically at 37°C for 48 hours (FIL-IDF method 149A-1997).

The *L. paracasei* PC201 lyophilisate count amounted to 7.0 x 10¹¹ CFU/g.

## Claims

1. Strain of *Lactobacillus paracasei* PC201 deposited at the Collection Nationale de Cultures de Microorganismes (CNCM) of the Pasteur Institute under number "CNCM I-4214" on July 28, 2009.

2. Strain according to claim 1, wherein said strain is in freeze-dried form.

3. Composition containing a strain of *Lactobacillus paracasei* PC201 according to claim 1 or 2 and at least one suitable carrier.

4. Composition according to claim 3, also containing other bacterial species, yeasts, prebiotics, vitamins, minerals, plant extracts and nutritional components.

5. Composition according to claim 3 or 4, in the form of powder, granulate, microgranulate, hard capsules, soft capsules, tablets, pills, liquid, drops, syrup, cream, gel, emulsion, lotion, spray, pessaries, yoghurt or dairy products.

6. Strain of *Lactobacillus paracasei* PC201 according to claim 1, for use as a medicament.

7. Strain of *Lactobacillus paracasei* PC201 according to claim 1, for use as a dietary supplement, food, food for a particular nutritional use, medical device, or cosmetic product, either for human or veterinary use.

8. Strain of *Lactobacillus paracasei* PC201 according to claim 1, for use in prevention and treatment or as adjuvant in the prevention and treatment of gastroenteric diseases and/or gastroenteric symptoms not attributable to disease.

9. Use of a strain of *Lactobacillus paracasei* PC201 according to claim 1 for the manufacture of a composition for the prevention and treatment or as adjuvant in the prevention and treatment of gastroenteric diseases and/or gastroenteric symptoms not attributable to disease.

10. Use according to claim 8, wherein the strain is administered concomitantly with or subsequently to an antibiotic treatment.

11. Use according to claim 8, for the prevention and treatment of intestinal dysbiosis.

12. Use according to claim 8, for the prevention and treatment of intestinal dysbiosis involved in the pathogenesis and/or progression of IBS (Irritable Bowel Syndrome).
